# EUROPEAN PATENT SPECIFICATION

(11) **EP 2 276 538 B1**
(45) Date of publication and mention of the grant of the patent: **20.03.2013**
(21) Application number: 09730286.3
(22) Date of filing: 24.03.2009
(51) Int. Cl.: A61M 39/26

(54) **Improvements relating to self-sealing connectors**
Verbesserungen in Bezug auf selbstabdichtende Verbindungselemente
Amélioration se rapportant à des connecteurs auto-obturateurs

(30) Priority: 09.04.2008 GB 0806440
(43) Date of publication of application: 26.01.2011
(73) Proprietor: Distribuidora Internacional De Medicamentos Y Equipo Médico, S.A. De C.V., Guadalajara JA 44190 (MX)
(72) Inventor: HAYES-PANKHURST, Richard Paul, Exeter EX5 5PJ (GB); LACY, Graham Keith, London SW12 8LE (GB); SWAN, Julian Francis Ralph, London W5 4XH (GB); MCLELLAN, Steven, London W13 9JZ (GB)
(74) Representative: Caldwell, Judith Margaret
(86) International application number: PCT/GB2009/050275
(87) International publication number: WO 2009/125212

(56) References cited:
- WO-A-96/00107
- WO-A-2006/013433
- WO-A-2006/088858
- US-A- 6 063 062
- US-A1- 2002 040 207
- US-A1- 2006 129 109

## Description

### Field of the invention

This invention relates to a self-sealing connector for establishing fluid communication between two fluid carrier lines and to a fluid administration system comprising said connector. The self-sealing connector is particularly suitable for use in an intravenous system.

### BACKGROUND OF THE INVENTION

### Description of prior art

It is known to provide a device to connect two tubes in order to deliver the fluid contents from a first tube into a second tube for subsequent delivery to a destination. Such a device enables the delivery of the fluid from the first tube to the destination to be flexible. For example, for drug, nutrient or blood delivery by intravenous line, fluid can be infused into a patient from a source via the connector, wherever the source is located, and without having to re-pierce the patient's vein each time infusion is necessary.

Due to problems encountered in the field of intravenous therapy over the last 25 years, various improvements have been made to medical connectors. From 1985 onwards medical connector design research was focused on the prevention of accidental needle stick injury to medical employees when manually connecting two medical lines of an intravenous system. The urgency to provide a solution to this problem was heightened by an increasing awareness of life threatening illnesses, such as AIDS. This led to the development of the first basic style of commercial needleless connector. However, the introduction of a needleless connector or valve between medical lines has created other problems in intravenous fluid delivery.

Currently, most of the commercially available connectors comprise several mechanical components in which one or more cavities are present between those components. Connectors in the prior art routinely comprise at least one space which is less easily accessible by fluid but, nevertheless, is still fluidly connected with the main fluid flow path. A connector is particularly undesirable if fluid passing through the connector is able to become trapped in a plurality of spaces. It is known that stagnant fluid promotes microbial activity, thus the presence of such spaces increases the risk that microbes will pass down the fluid line and the patient will become infected. Bacteremia associated with infusion therapy is known to increase morbidity and prolong patient hospitalization. Since intravenous therapy is among the most widely used invasive procedures in all health care settings, it is essential that the risk of infection via medical connectors is minimised.

In WO9721464 the inventor attempts to solve the problem of fluid retention within the spaces in the connector by providing 'fluid escape spaces' between a resilient member and housing of the valve. When the resilient member is compressed during connection, fluid trapped in the spaces is forced from the connector. This solution is not ideal as the fluid still has access to additional spaces within the connector. It will not completely prevent the possibility that some fluid may get trapped or blocked within the fluid escape spaces. It is also possible that microbes external to the connector may be able to access the interior of the connector via the fluid escape spaces. Further still, this design suffers the disadvantage that a volume of the fluid may be lost from the fluid flow passage at any time, and as a result, dosing may be affected.

In a further attempt to reduce the risk of microbial transfer it is desirable to provide a surface which is easily capable of being disinfected quickly; typically by wiping the connection surface with a swab. It is also an advantage to provide a connector which self-seals as this reduces the possibility of microbial transfer to the interior of the connector. Thus, many connectors of the prior art, for example: WO 2004/103454 (Alaris Medical Systems), include a swabable seal or septum having a slit therein, the seal being positioned over a pin such that the pin, and hence the positive flow path, can only be accessed by deforming the slit into an open position or pushing the septum over the pin using downward force, e.g. from a nozzle member. The seal or septum provides a flat swabable surface which can be easily wiped and which covers or protects the pin from external microbes. However, such designs suffer the major disadvantage that space is often present somewhere between the seal component and the pin component. As a result fluid can be displaced as will be further described below.

US 2002/0040207 (Lopez) is another example of a medical valve where a compressible seal surrounds a spike having at least one hole located near its tip, the seal being adapted to be moved into a compressed state upon insertion of the tip of the medical implement.

In US 6,063,062 (Paradis) the valve comprises a stationary probe within a housing, the probe extending above the surface of a surrounding seal member which itself is depressed, rather than compressed, upon application of pressure from the blunt end of a syringe. The raised tip of the probe provides helps location of the male Luer element during connection of the line.

The connector designs in the prior art typically comprise two luer elements: a female component and a male component; releasably locked together by the use of a standard luer thread lock. At least one of these components requires a plurality of different internal mechanical features to actuate a valve and activate fluid flow between the two elements when they are locked together. However, the mechanical features add 'dead space' to the main fluid pathway. The presence of the dead space causes a volume of the fluid to be displaced in the downstream direction

(positive bolus), or in the upstream direction (negative bolus or suck-back) when the connector is activated and/or deactivated.

In the simplest of examples, a patient may receive an incorrect dosage of drug due to the displacement of fluid during connection/disconnection to the intravenous infusion line. It is conceivable that where a male luer nozzle is used as the actuator, that the volume of fluid displaced during actuation will be at least approximately equal to the volume of the male luer nozzle penetrating into the female luer when they are coupled together. This volume and direction of displacement can vary substantially, based upon device components and materials used etc.

Uncontrolled fluid displacement during intravenous therapy is extremely disadvantageous and potentially dangerous. For example, in the case where the drug to be administered is a potent one, the patient must receive an accurate dose in accordance with a pre-calculated window of upper and lower limits. Typically, the upper limit will be only marginally above the dose required for efficacy, to prevent unwanted side effects. Administration of a dose which is outside the pre-calculated upper and lower limits may result in a reduced therapeutic effect, or a toxic effect, to the patient.

Accordingly, the invention arises from the Applicant's efforts to provide a self-sealing connector in which microbial activity is not promoted and which provides negligible positive and negative displacement of fluid. It is further intended to achieve these objectives whilst providing a connector which at least maintains an acceptable flow rate but, more preferably, has an optimised flow rate. It is further intended to achieve these objectives whilst providing a connector which is easily disinfected before and after use.

### SUMMARY OF THE INVENTION

In the present invention a self-sealing fluid line connector is provided, the connector comprising: a housing including a neck; a base; a substantially hollow pin extending up through said base into said housing, the hollow pin having a tip portion and at least one aperture; and a resilient member mounted about said pin, the resilient member having a sealing end for sealing the at least one aperture, wherein a surface of the sealing end and a surface of the tip portion of the pin together defining a planar receiving surface such as for a nozzle, and the sealing end of the resilient member further includes a flange seal adapted to maintain sealing contact with an inner surface of the housing neck both when the resilient member is in a first position and the at least one aperture is sealed and when the resilient member is compressed to allow fluid to flow through the at least one aperture.

By "sealing the at least one aperture" it is meant that the aperture is isolated from the external environment. The sealing may be directly over or around the aperture as later described.

The Applicant has recognised that overcoming the problems of significant fluid displacement and microbial contamination are possible by creating a mechanically simple connector in which the components cooperate such that there is very little dead space into which fluid may be displaced before, during or after actuation.

Additionally, the flange prevents fluid from accessing space between the housing and the resilient member. The flange remains in contact with an inner surface of the neck housing when the resilient member is relatively non-compressed. Hence, the risk of fluid passing into undesirable spaces within the connector is further reduced minimising the possibility that such spaces in the connector will be contaminated.

In the first position, typically in its substantially non-compressed state, the sealing end of the resilient member encloses the at least one aperture so that it is sealed. The end surface of the tip portion of the pin and a surface of the sealing end of the resilient member together form the receiving surface for receiving an in-flow nozzle, syringe nib, tubing connector or equivalent. Advantageously, therefore, the connector of the invention does not require an additional seal or cover to enclose the pin in order to protect it from environmental microbial contamination.

As the connector of the invention also prevents loss of fluid there is an additional advantage that the actual dosage supplied to the patient is maintained within the acceptable limits of the pre-calculated dosage.

When the resilient member is in a relatively non-compressed form, namely the first position, the sealing end of the resilient member is adapted to surround the tip portion of the pin. Preferably, the sealing end has an inner surface which contacts the tip portion of the pin.

In one embodiment, a top surface of the tip portion of the pin and a top surface of the sealing end of the resilient member may together define a substantially unbroken receiving surface. The unbroken receiving surface is achieved by providing a resilient member in which the sealing end has an inner surface which surrounds and contacts an outer surface of the pin's tip portion at least at its outermost end. The inner surface of the resilient member essentially forms a seal with the tip portion.

In another embodiment, the sealing end may hug the tip portion along substantially its entire length, or at least to just below the or each aperture, such that there is no space therebetween. This is particularly useful as external contaminants are prevented from accessing the internal space of the connector.

It is recognised by the Applicant that by providing a substantially continuous seal along the length of the sealing end of the resilient member around the pin tip, there is a risk that friction is created between the contacting surfaces of the pin, resilient member and housing, making it difficult to move the sealing end of the resilient member down over the pin, which is undesirable.

Therefore in another embodiment, the resilient member is adapted to contact the surface of the pin and the housing at a location above or below, preferably above and below, the least one aperture of the pin to seal it when in the first position. For example, the resilient member preferably comprises at least one (such as one, two, three or more), and more preferably two inwardly directed radial flanges, e.g. in the form of ringed sections or sealing rings, which create a local seal around the at least one aperture of the pin. The sealing end therefore provides an interference fit around one or more portions of the pin tip rather than fitting tightly around substantially the whole length of the pin tip in order to seal the aperture(s).

This is particularly advantageous because when the resilient member is compressed, the frictional force, due to limited contact between the outer surface of the pin and between the inner surface of the housing, is relatively low. When the device is connected, the resilient member is able to slide down the shaft of the pin with ease. Further, when the connection is terminated, the resilient member returns to its non-compressed state without hindrance and a seal is re-formed around the at least one aperture of the pin.

The flange of the resilient member serves as a barrier to prevent contaminants entering the connector between the housing and the seal. However, it will be appreciated that such a contact area is relatively small and therefore this feature does not significantly increase the friction between the surfaces of the resilient member and the pin during use.

The housing may further comprise an external ledge or barrier. Usefully, when the connector comprises a screw thread arrangement or the like, the ledge prevents a corresponding screw thread of the in-flow nozzle, or the like, being over-rotated on to the connector. It is possible that over-rotating the screw thread could damage the connector and/or the in-flow nozzle being used, which is undesirable.

It is perceived that any conventional means enabling the housing and/or base to be secured to fluid carriers could be utilised. The invention is not limited, therefore, in its broadest sense to standard screw thread arrangements. In some embodiments, it is preferred that there is no male luer lock (screw thread) on the base, but instead a cylindrical end for securely fitting with tubing.

When an in-flow nozzle, or the like, is positioned on to the receiving surface and pressed downwardly in the longitudinal direction of the connector to make a fluid connection with the aperture of the hollow pin, the resilient member is compressed and deforms such that the sealing end is able to move over the tip portion of the pin and the at least one aperture. By virtue of the flange, at least part of the sealing end remains in sealing contact around the tip portion during compression of the resilient member. Accordingly, the connector suffers negligible fluid displacement during use as the novel arrangement of the parts in the connector allows for very little dead space therein.

Effectively the only 'accessible' dead space present in the connector is the volume defined within at least one aperture of the pin and is determined by the depth of a wall of the pin and the size of the aperture therein. The volume of fluid displaced during use of the connector is, therefore, partly determined by the depth and size of the at least one aperture in the wall of the hollow pin.

It is preferable to position the one or more apertures proximal to the tip portion and more preferably, directly beneath the tip portion. Such an arrangement does not overly affect the flow rate of fluid through the connector and the hollow pin is simple to manufacture.

While the pin of the connector may have a single aperture, it may be useful to have at least two apertures in the pin as this promotes a faster flow rate of fluid through the connector. Each extra aperture marginally increases the amount of dead space in the connector (depending on the size of the aperture) and thus may proportionally increase the total volume of fluid that will be displaced during use. When the hollow pin of the connector has two apertures, these are preferably positioned substantially opposite one another, substantially perpendicular to the shaft of the pin. This particular arrangement is also advantageous because in a method for manufacturing this connector it may be easier and simpler to form opposing apertures.

On the other hand, the inventors have surprisingly found that three apertures in the pin optimizes the flow rate without significantly affecting the amount of fluid that is displaced positively (on connection) or negatively (on disconnection) through the connector. Accordingly, in one embodiment, the pin has three apertures, evenly spaced around the circumference of the shaft of the pin.

As well as the number of apertures provided, the specific geometric configuration of the pin's aperture(s) also has an effect on the flow rate and the fluid displacement characteristics of the connector. In the simplest arrangement, the at least one aperture has a substantially circular cross-section to promote flow of fluid through the connector.

However, it is preferred that the pin's apertures are profiled to complement the pin's shaft. That is, rather than being perpendicular (at a 90 degree angle, or thereabout) to the shaft, they preferably branch out from the shaft (obliquely through the wall of the pin), for example at an angle of at least 100 degrees or at another obtuse angle. Since the fluid flowing through the connector must temporarily re-direct in order to enter or exit the pin shaft, increasing the internal angle of the apertures relative to the main bore or shaft of the pin results in reduced fluid resistance. Following the initial connection, the fluid passing through the connector follows a relatively non-tortuous pathway. For example, due to the improved internal geometry of the pin, a relatively limited surface area of the pin's internal surfaces are contacted by the fluid and therefore the back pressure experienced in the connector is reduced. It has shown during testing that the turbulence of the fluid flow is minimised which improves the flow rate.

To enhance the effect described above, where the tip portion is solid, an internal ceiling of the pin's shaft, e.g. the internal surface of the tip portion, is preferably substantially non-flat, in particular, rounded, bulbous or pointed. For example, if the fluid passes up through the shaft towards the connecting device, e.g. a nozzle, fluid that contacts a rounded ceiling will be more easily re-directed through the angled apertures. Usefully, by having an internal ceiling with a profile which facilitates the fluid flow between the apertures and the shaft of the pin, flow rate is maximised.

For intravenous administration, it is preferred that the diameter of each aperture does not exceed 2mm. The one or more apertures may be elongated, e.g. a 2:1, length: width ratio, the length being parallel with the longitudinal axis of the connector. Such a configuration further increases the maximum flow rate of fluid through the connector.

It is envisaged by the Applicant that, in other medical-type applications, the general size of the aperture may be scaled according to the dimensions of the connector, as is required to achieve a desirable flow rate. In particular, where the connector is made larger or smaller for use in alternative applications, e.g. engineering or micro-fluidics.

In one embodiment, the tip portion of the pin is solid. Advantageously, this provides additional rigidity to the structure of the pin.

It is preferred that the tip portion is made integral with the hollow pin. However, it is possible that these components can be manufactured separately and the tip portion fixed to the shaft thereafter.

When a nozzle is pressed downwardly on to the receiving surface and fully engages with the connector, it is essential for the fluid to be able to flow from the opening in the nozzle and through the at least one aperture to the pin. Therefore, when the nozzle and the connector are engaged, there is a space between the pin and an interior surface of the insertion nozzle. The size of the space is defined by the diameter of the tip portion of the pin relative to the interior diameter of the nozzle. It is preferred that this space is as small as possible to reduce the volume of fluid able to be displaced but still allows an adequate flow of fluid.

It is preferred that the length of the sealing end of the resilient member extends from the end of the pin, where it partly defines the connector's receiving surface, to below the aperture in the pin. When the resilient member is compressed, the length of the sealing end contacts the shaft of the hollow pin below the aperture. Advantageously, this creates a circumferential seal around the pin's shaft and prevents fluid loss between the pin and the resilient member.

The housing comprises a neck which extends round the sealing end of the resilient member. In one embodiment the neck of the housing and the sealing end of the resilient member are substantially the same length.

The positioning of the resilient member relative to the housing and pin may be enhanced. In particular, the housing may further comprise an internal stop arranged to abut against the resilient seal to accurately position it when in the first position, i.e. a relatively non-compressed state.

The resilient member may comprise a shoulder for positioning the resilient member relative to the housing and the pin. For example, when the resilient member is in a substantially non-compressed state, the shoulder abuts an interior surface of the housing to prevent the sealing end from moving too far into the neck. Specifically, the shoulder accurately positions the resilient member such that the upper surface of the sealing end is aligned with the upper surface of the tip portion of the pin to create the receiving surface.

Furthermore, when the connector has a resilient member sealing end having sealing rings, the shoulder further serves to position accurately the two sealing rings relative to the pin's at least one aperture, e.g. above and below the aperture(s). Usefully, this helps ensure that the aperture(s) is sealed when the resilient member is in the first substantially non-compressed state.

The resilient seal may typically comprise a section contractible in a longitudinal direction which allows the resilient member to compress. Such a section may comprise one or more corrugations. Where a plurality of corrugations is used they may have generally the same shaped cross-section and/or diameter, but it is possible they may have a varied cross-section and/or different diameter to one another.

Preferably, the neck of the housing is shaped to enable a nozzle to be guided into the connector. The neck of the housing may therefore have a chamfered or angled edge to assist. This allows the clinician to more easily locate the receiving surface (defined by the upper surfaces of the sealing end and the tip portion of the pin) with the nozzle of a syringe, or the like. More preferably, the neck comprises a guiding surface which slopes inwardly towards the receiving surface. This arrangement provides the advantage that the receiving surface is able to be located quickly for cleaning, e.g. by swabbing, to reduce or prevent the possibility of microbial contamination from the environment. Usefully, since the clinician is always correctly guided to the receiving surface, the risk that the nozzle will be misplaced and ultimately that the pin will be damaged is reduced.

The resilient member may include a foot portion, or supporting flange, at an opposite end to the sealing end, that rests on the base of the housing.

When the resilient member is compressed, there is a minor risk that the contractible section (corrugations) will contact and stick to the inside of the housing. This is undesirable since, following removal of a syringe, the resilient member may be prevented from returning to a sealed position. The supporting flange may therefore comprise a channel for equalising the air pressure between the inside and the outside of the resilient member when the resilient member is compressed.

In a preferred embodiment the housing or the base, more preferably the base, includes a recessed section for accommodating the lower portion of the resilient member. The foot portion, or supporting flange, nests in and is supported by the base of recess. The resilient member extends upwardly from the base, surrounding the pin. Advantageously, the resilient member does not require any further means to be secured within the connector. This arrangement simplifies the number of components within the connector and simplifies the manufacturing method, hence reducing the manufacturing cost per connector unit.

In a preferred arrangement, the pin and base are integrated as a single component of the connector. Usefully, the self-sealing connector may therefore comprise only three component parts, i.e. a housing, a base comprising the pin and a resilient member. This modification of the connector further simplifies the manufacturing process and manufacturing cost per connector unit. Preferably, the housing substantially surrounds the resilient member and the resilient member substantially surrounds the pin.

In a particular embodiment, the base may define a first passage, such as a cylindrical pipe, for connecting with a tubing or similar fluid transport means. Usefully, a screw thread/luer lock is not required at the base. The base may be completely integrated with the pin such that the pipe itself defines a lower shaft (part of the shaft which extends down from the base) and therefore defines together with the shaft of the pin a fluid pathway through the connector. Therefore, in this arrangement, the pin itself does not define the lower shaft.

In a preferred form, the base further defines at least a second passage which extends as a branch from the first passage. The branch is additional to the first passage and may be fitted to a section of tubing or the like, independent to the tubing connected with the first passage. Usefully, this feature provides that the fluid pathway running through the connector is directly accessible via the branched passage without disrupting an existing connection at the first passage. For example, while the connector is already engaged with a nozzle at the pin end and tubing at its first passage, a tertiary connection can be made at its second passage and another fluid may be introduced into the lower shaft of the connector. The second passage is preferably arranged at an oblique angle with respect to a longitudinal axis of the connector (the second passage pointing generally towards the pin end of the connector). It is envisaged by the inventors that the base could be further adapted to define more than two passages each having the same or different commercially desirable shape and size.

It is intended that the invention includes a connector as described above with any combination of non-conflicting features described in the above embodiments.

The invention extends to a fluid line system comprising a connector as described in any of the embodiments above, alone or in combination, and further, to a medical administration system or an intravenous system comprising said connector.

The invention also encompasses a method for connecting two fluid lines as described in any of the previous embodiments.

### BRIEF DESCRIPTION OF THE DRAWINGS

In order that the invention may be more readily understood, reference will now be made, by way of example, to the accompanying drawings in which:
Figure 1 is a cross-sectional view of a connector of the invention where the resilient member is in a first relatively non-compressed state and an aperture is sealed;
Figure 2 is a cross-sectional view of the connector of Figure 1 where the resilient member is compressed and the aperture is unsealed;
Figure 3 is a cross-sectional view of a connector of an embodiment of the invention where a resilient member is relatively non-compressed and an aperture is sealed:
   Figure 3 (a) is a cross-sectional view of a connector according to a further embodiment of the invention where a resilient member is relatively non-compressed and an aperture is sealed;
   Figure 3 (b) is an enlarged view of the connector of Figure 3a;
Figure 4 is a cross-sectional view of the connector of Figure 3, where the resilient member is compressed and the aperture is unsealed:
   Figure 4 (a) is a cross-sectional view of the connector of Figure 3a and 3b, where the resilient member is compressed and the aperture is unsealed;
Figure 5 is a sectioned perspective view of a variant of the pin of the connector:
   Figure 5 (a) is an enlarged perspective view of the pin tip of Figure 5;
Figure 6 is an exploded view of a connector in accordance with another embodiment of the invention:
   Figure 6 (a) is an alternative view of the connector shown in Figure 6;
Figure 7 provides three perspective views (a), (b) and (c) of an assembled connector according to a conceptual visualisation of the invention;
Figure 8 provides three different views (a), (b) and (c) of a preferred embodiment of the connector in which the base comprises first and second passages;
Figure 9 shows two perspective views (a) and (b) of the connector of Figure 8; and
Figure 10 shows two additional cross-sectional views (a) and (b) of the connector shown in Figure 8, before and during its use.

### DETAILED DESCRIPTION OF THE INVENTION

Where dimensions are given in this specific description, they are given only for the purpose of illustration and are not intended to limit the invention in its broadest sense.

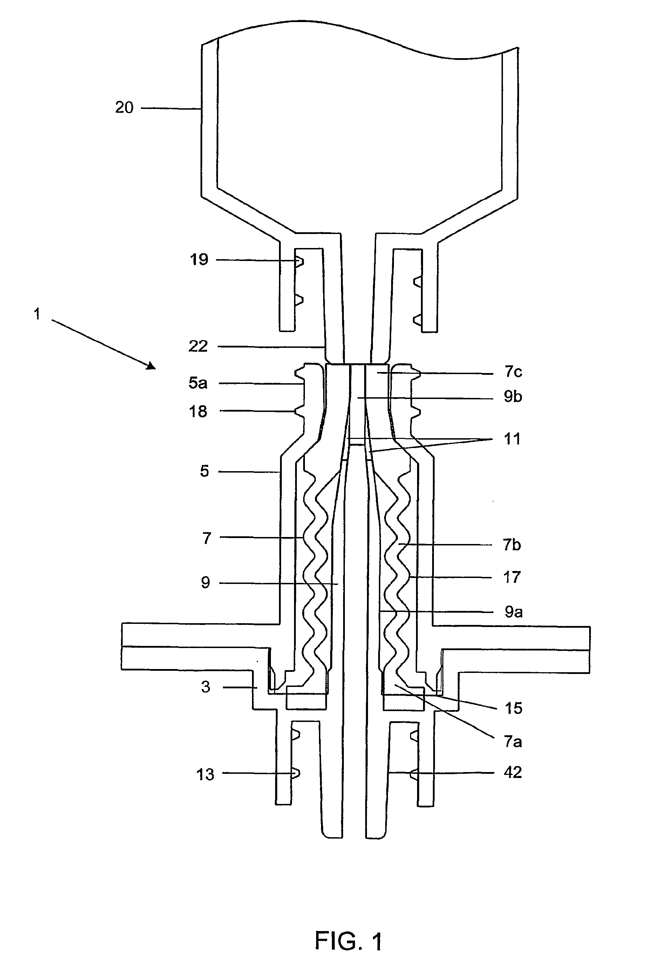

Figure 1 shows a connector 1 in an unengaged state, comprising a base 3, a housing 5; a resilient member 7; in a first position which is in a relatively non-compressed state and a substantially hollow pin 9.

The base 3 comprises a base screw thread 13 for releasably securing the connector to a first fluid carrier (not shown) via a corresponding screw thread (not shown) associated with the first fluid carrier. The first fluid carrier may be a means for further transporting the fluid to another destination or a port, either of which access a medical system or apparatus. A substantially hollow pin 9 comprises a hollow shaft 9a that extends through the centre of base 3. A relatively short length 42 of the shaft 9a extends below the base 3 and is engagable with the first fluid carrier when the base screw thread 13 and fluid carrier screw thread are in a mutual locking arrangement.

A relatively longer length of the hollow shaft 9a extends above the base 3 gently tapering at its distal end. The pin 9 further comprises a tip portion in the form of a solid cylindrical member 9b that sits on the tapered end of the hollow shaft 9a, further extending the length of the pin 9. The cylindrical member 9b is integral with the hollow shaft 9a. The tapered surface of the pin 9 comprises at least two opposing apertures 11 immediately beneath the cylindrical member 9b. The geometric configuration of the apertures 11 may vary considerably to balance the flow rate and fluid displacement characteristics of the connector 1, as will be explained in further detail below.

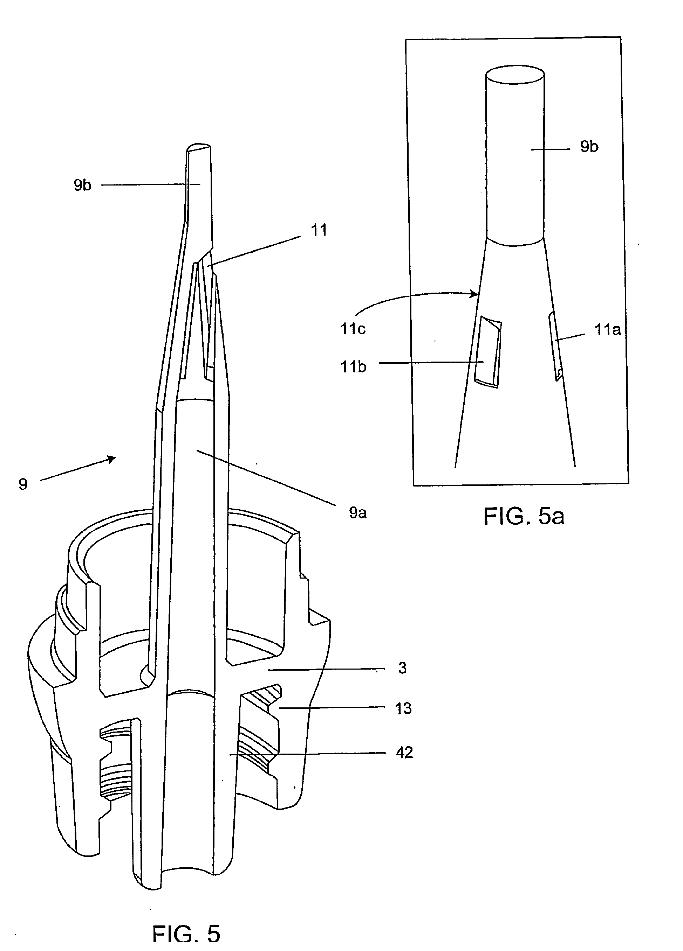

The geometric cross-section of the interior wall of pin 9 may vary along the length of the shaft 9a to ensure that, in use, there is a suitable balance between the flow rate of fluid down the hollow shaft 9a and the rigidity of the whole structure of the pin 9. This will be further explained in the description of Figure 5 below.

Base 3 further comprises a supporting arrangement in the form of a recess 15 in which a bottom end 7a of the resilient member 7 rests for support. The resilient member 7 comprises a longitudinally contractible section 7b, which is flexible and resilient in a longitudinal direction; and a sealing end 7c. Flexibility is imparted to the resilient member 7 by a series of annular corrugations 17 comprising a succession of parallel alternating ridges and grooves. The corrugations 17 extend completely around the shaft 9a of the pin 9 and partway along its length. The number of corrugations 17, their length and their depth can be selected to suit the size and application of the connector 1. In particular, the annular corrugations 17 in this embodiment all have substantially the same sized diameter. The sealing end 7c is made of a resilient material, for example silicone, such that on compressing the resilient member, the sealing end 7c expands radially in order to slide over the increasing diameter of the pin shaft 9a.

The sealing end 7c is integral with the longitudinally contractible part 7b, its length extending in a longitudinal direction. The sealing end 7c extends completely around the cylindrical member 9b and the apertures 11 in the hollow shaft 9a. The apertures 11 are completely sealed by the sealing surface 7c. A top end of the sealing end 7c, lying in the same plane as the top end of the cylindrical 9b, defines a surface for receiving an in-flow nozzle.

The housing 5 comprises a cylindrical neck portion 5a which extends completely around the sealing end 7c of the resilient member 7. Neck portion 5a includes a screw thread 18 for releasably securing the connector to a second fluid carrier 20 via a corresponding screw thread 19.

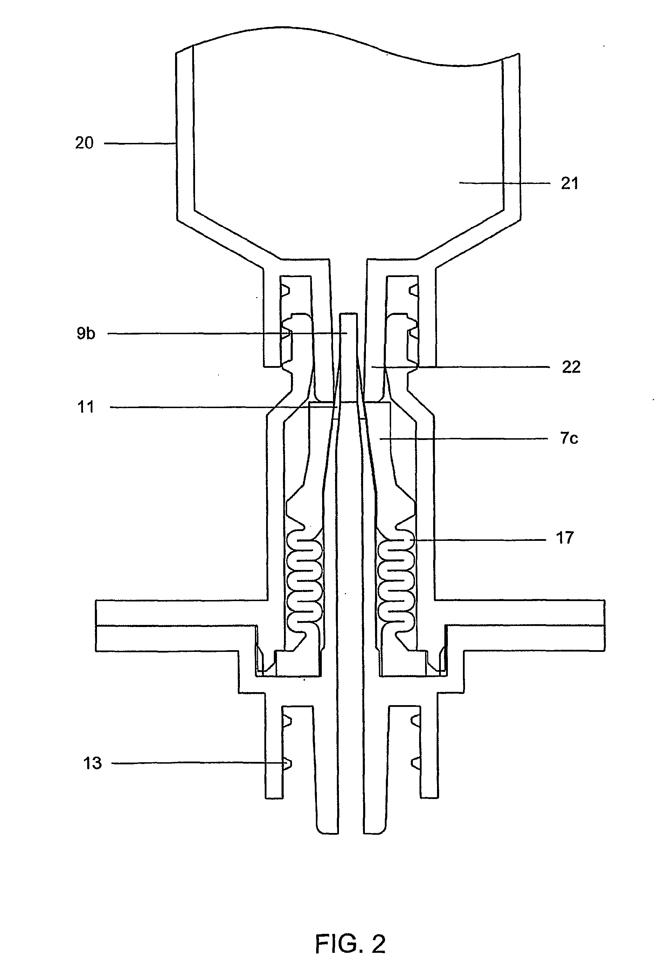

Referring now to Fig 2, the connector 1 is shown fully engaged with a nozzle 22 of second fluid carrier 20. The nozzle 22 is initially pressed downwardly to contact the connector's receiving surface that is defined by the tip portion 9b of the pin 9 and the sealing end 7c of the resilient member 7. The nozzle 22 is moved further downwardly into the connector in a longitudinal direction of the connector by engaging the screw threads 19 of the second fluid carrier with corresponding threads 18 of the neck housing 5a of the connector 1. As the nozzle 22 moves downwardly, the sealing end 7c is forced to slide over the cylindrical member 9b, and corrugations 17 in the contractible section 7c compress.

The threads 18 and 19 are further screwed together until they mutually lock in place thereby securing the second fluid carrier 20 to the connector 1. This motion drives the nozzle 22 further downwards into the housing 5, pushing the sealing end 7c and causing the contractible section 7b to become fully compressed. In this arrangement the cylindrical member 9b sits upwardly within the nozzle 22. The apertures 11 are no longer sealed by the surface of the sealing end 7c and fluid 21 contained in second fluid carrier 20 is able to flow from the nozzle 22 through apertures 11 and into the shaft 9a of the pin 9.

The base 3 of the connector 1 is securely connected with a first fluid carrier (not shown) via screw thread 13 or other means prior to engagement with the second fluid carrier 20. Accordingly, once the second fluid carrier 20 has fully engaged with the connector 1 and fluid 21 flows from its nozzle 22 and along the shaft 9a of pin 9, it may continue to flow directly into the first fluid carrier. In this engaged state, the connector 1 enables fluid to pass freely through in either a positive direction towards the first fluid carrier, and for example, to a patient; or in a negative direction towards the second fluid carrier 20.

The connector 1 has minimal space in which fluid passing through the connector is retained after the connector 1 has been utilised to deliver fluid from the second fluid connector 20 into the first fluid connector. When the nozzle 19 is disengaged from the connector 1 the fluid is only displaced into the space between cylindrical member 9b and the sealing end 7c and/or the space defined by the depth and size of the at least one aperture 11 in the wall of the hollow pin shaft 9a.

The inventors have demonstrated experimentally that the volume of fluid displaced is about 0.005cc. Accordingly, positive bolus supplied to the first fluid carrier on reconnection of the connector is limited to a negligible value.

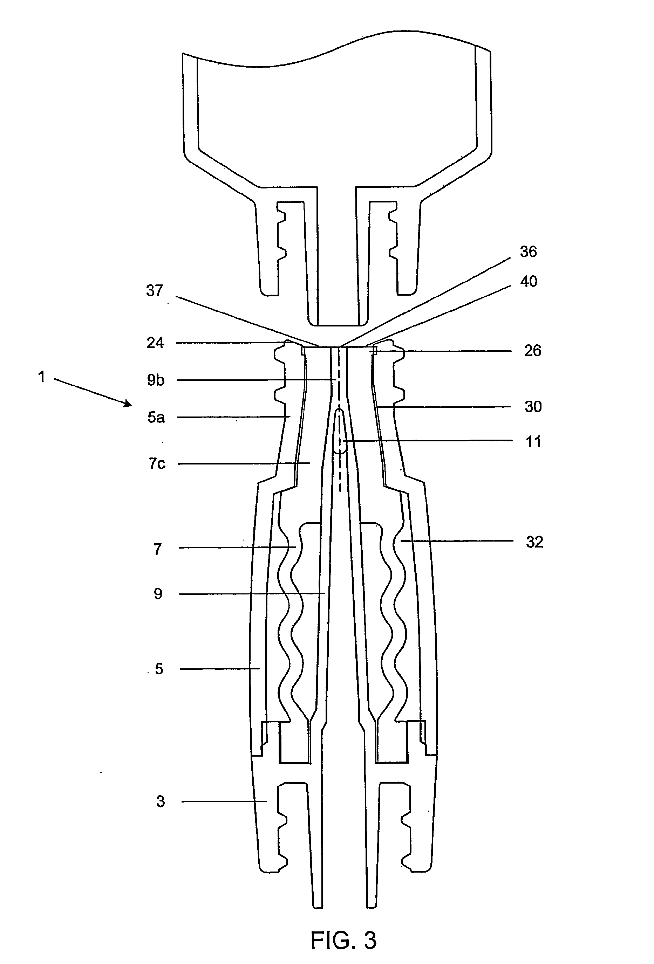

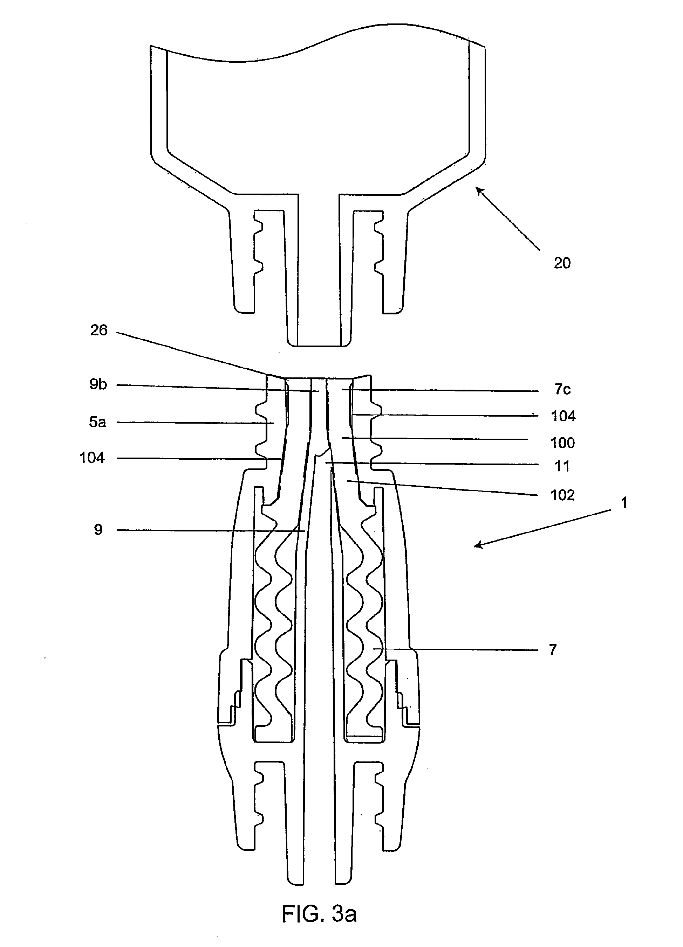

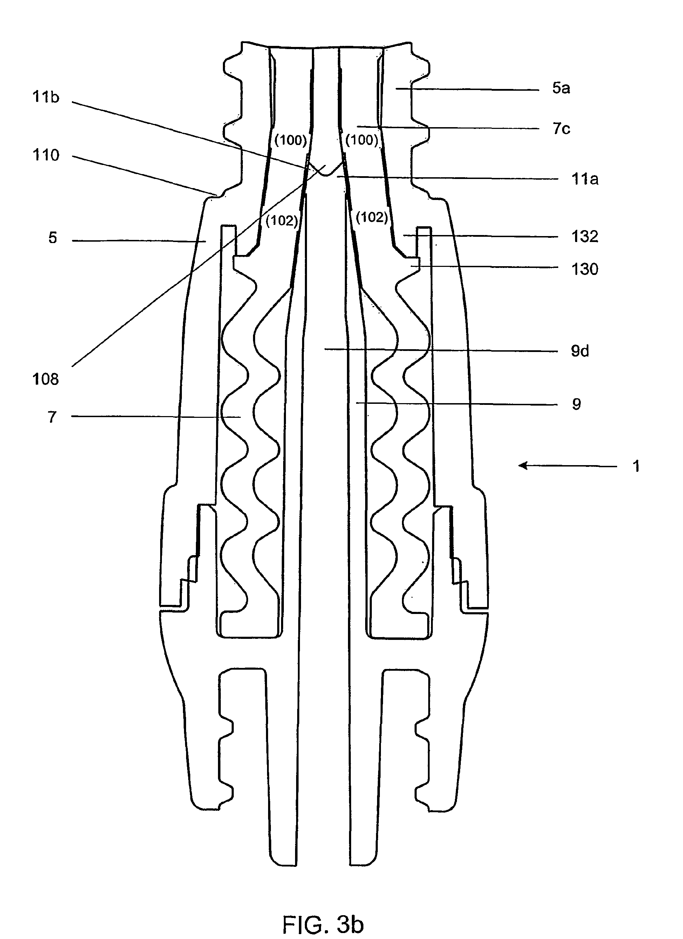

Figure 3 shows a variant of the connector 1 in Fig 1. The main components of the connector, i.e. the base 3, the housing 5, the resilient member 7 (in a relatively non-compressed state) and the substantially hollow pin 9 are generally arranged as previously recited in the description of Figure 1. In this embodiment there are variations to the components each of which provide an additional advantage.

The neck of the housing 5a which circumferentially surrounds the tip portion 7c of the resilient member comprises an upper surface 24 which is upwardly inclined away from the plane of the receiving surface, defined by the upper surfaces 36, 37 of cylindrical member 9b and tip portion 7c. The inclined surface 24 aids the nozzle 22 of the fluid carrier 20 to be guided more easily on to the receiving surface. Thus, in a clinical setting the receiving surface of the connector 1 can be effortlessly located and the connection process is efficient.

The tip portion 7c of the resilient member 7 comprises a circumferential flange 26, which abuts an inner surface of the neck of the housing 5a. The flange 26 provides a barrier to spaces 30 and 32 between the neck of the housing 5a and the resilient member 7, thereby reducing the risk that microbes will contaminate the connector in its unengaged state. Further, if any fluid is present on the receiving surface following disengagement of the connector it will not be able to pass into the space 30. The inclined surface 24 will also retain excess fluid on the receiving surface until it has been swabbed and the fluid is completely removed from the connector 1.

Cylindrical member 9b has an upper surface 36. An inner surface of the seal end 7c tightly hugs cylindrical member 9b. Thus, the connector's receiving surface 40, as defined by the upper surface 36 of cylindrical member 9b and an upper surface 37 of seal end 7c. Any contaminants and fluid present on the receiving surface 40 are prevented from moving in between the cylindrical member 9b and the sealing end 7c. The risk of passing microbes to the patient via the connector 1 is minimised as there is no space between cylindrical member 9b and the total dead space within the connector 1 is minimised. This further reduces the volume of fluid that may be displaced during disconnection and/or reconnection of the connector.

Figure 3a shows a preferred embodiment of the connector 1. The general arrangement of the connector 1 is as previously described in Figure 3 but is additionally provided with a further number of advantageous features, each of which will be described below in detail.

The sealing end 7c of the resilient member 7 has first and second sealing contacts in the form of sealing rings 100, 102 which press against an outer surface of the pin 9 and an inside surface of the neck of the housing 5a. The two sealing rings 100, 102 are arranged above and below three apertures (one shown for simplicity) 11 of the pin 9 to create a dual seal there around when the resilient member 7 is in a relatively non-compressed state. As the sealing rings 100, 102 together provide a local seal around the aperture 11, an airspace 104 exists between the neck of the resilient member 7c and between the pin 9. These airspaces are defined by the flange 26 and the first sealing ring 100 or the first sealing ring 100 and the second sealing ring 102. The airspaces 104 reduce friction created in the connector 1 as a result of contact between the surfaces of the sealing end 7c and the housing neck 5a or the pin 9.

The specific arrangement of local sealing rings 100, 102, with airspaces 104 located there between provides at least two significant advantages: a reliable seal is maintained when the connector is not engaged with a nozzle of a fluid carrier (the non-compressed state) and movement of the sealing end 7c about the pin 9 is facilitated during engagement with the nozzle of the fluid carrier (to move in and out of the compressed state).

Figure 3b is an enlarged view of connector 1 of Figure 3a in which the pin 9 has two apertures 11 a, 11 b (for ease of view to are depicted, but the preferred embodiment has three) each of which branch out from the internal bore 9d of the pin 9 at an obtuse/oblique angle. The fluid flowing through the connector 1 will need to temporarily re-direct in order to enter or exit the pin 9, thus the shallow internal angle of the apertures 11a, 11b relative to the internal bore 9d will serve to reduce fluid resistance. An internal ceiling 108, which in this embodiment is located at the base of the internal surface of the cylindrical member 9b of the pin 9 is moulded substantially according to the desired profiles of the two apertures 11a, 11b. The ceiling 108 is rounded off to facilitate the flow of incoming/outgoing fluid to (or from) the angled apertures 11a, 11 b from (or to) the internal bore 9d.

The housing 5 additionally comprises an external ledge 110 for preventing the corresponding screw thread of the in-flow nozzle or something alike (not shown) being over-rotated on to the connector 1 and causing damage to the apparatus.

The resilient member 7 has a shoulder 130 which accurately positions the resilient member 7 relative to the housing 5 and the pin 9. When the resilient member is in a substantially non-compressed state, the shoulder 130 preferably positioned at the base of the sealing end 7c of the resilient member 7, abuts an interior surface of the housing 5 preventing the sealing end 7c from moving too far into the neck of the housing 5a. Furthermore, the shoulder 130 positions accurately the two sealing rings 100, 102 relative to the pin's apertures 11a and 11 b to ensure that the apertures 11 a and 11 b are sealed. The positioning of the resilient member 7 relative to the housing 5 and pin 9 is enhanced by an internal stop 132 which abuts the shoulder 130 of the sealing end 7c.

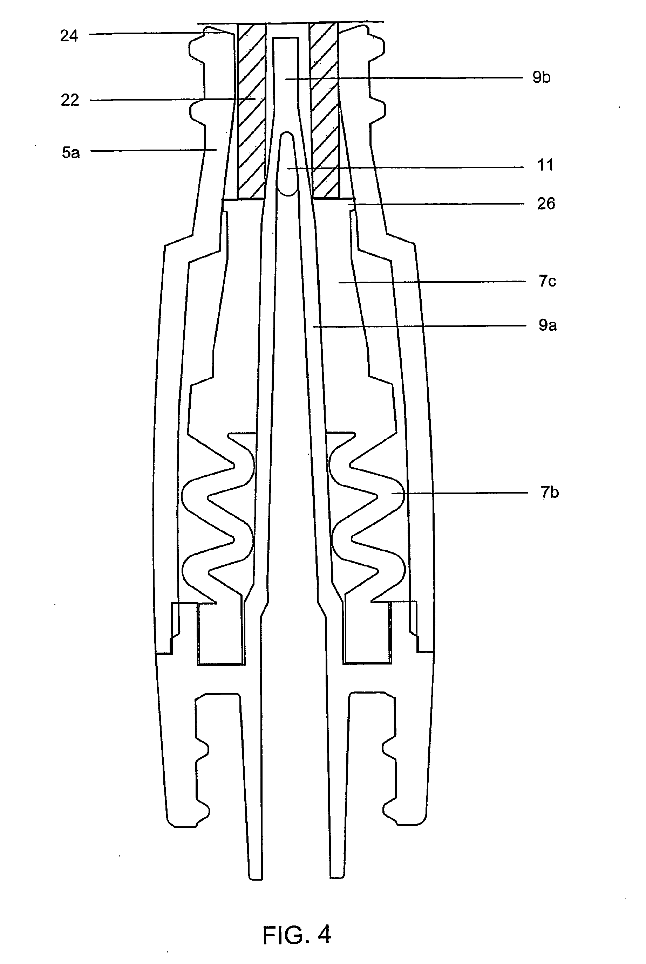

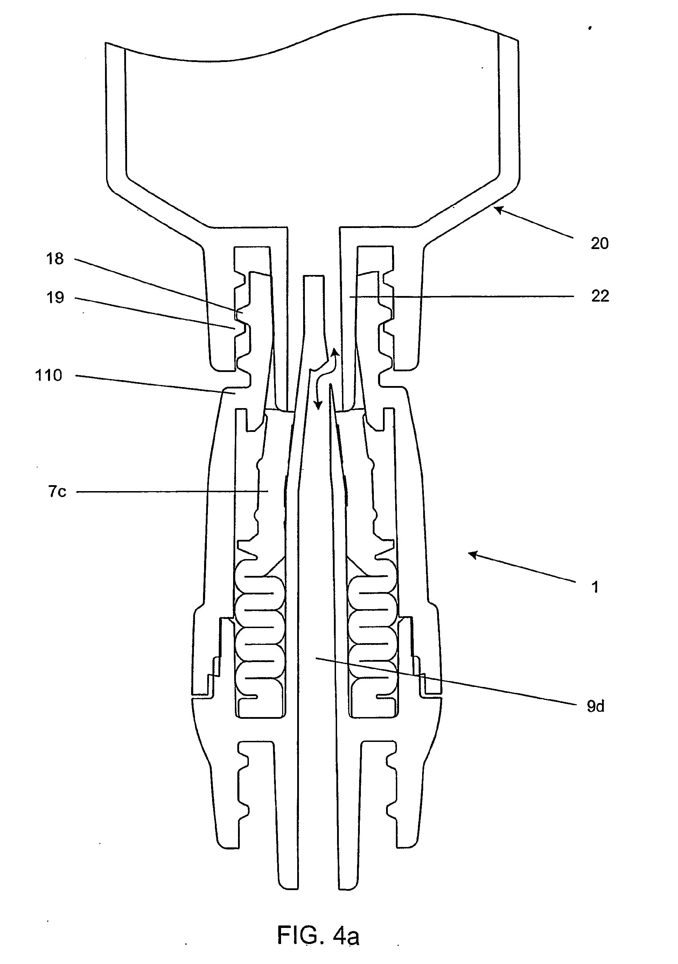

The above-described connector 1 is shown again in Figure 4, this time engaged with the nozzle 22 of the second fluid carrier 20. The first 20 and second (not shown) fluid carriers are secured to the connector 1 as previously detailed in the description of Figure 2. In this embodiment nozzle 22 has been guided by the inclined surface 24 of the housing on to receiving surface 40 of the connector. The nozzle 22 presses the sealing end 7c over the cylindrical member 9b and aperture 11. The distance of the movement of the sealing end 7c is somewhat limited by the longitudinally contractible section 7b that temporarily compresses and shortens in length. The housing neck 5a and the resilient sealing end 7c have substantially equal lengths. Accordingly, the flange 26 of the sealing end 7c remains in contact with a part of the inner surface of the neck housing 5a before, during and after the resilient member 7 is compressed. Thus, regardless of whether the resilient member is in a compressed or relatively non-compressed state, the flange 26 is always suitably positioned to prevent fluid from passing to undesirable space 32 in the connector. This serves to prevent a microbial presence within the connector.

In Figure 4a the embodiment of the connector shown in Figures 3a and 3b is engaged with the nozzle 22 of the second fluid carrier 20. The first 20 and second (not shown) fluid carriers are secured to the connector 1 as previously detailed in the description of Figure 2. In this embodiment the distance of the movement of the sealing end 7c is further limited by the external ledge 110 of the housing 5 which also prevents the screw thread 19 of the in-flow nozzle 22 over-rotating with the counter screw thread arrangement 18 on the connector 1. Following the initial connection, the fluid may flow through the internal bore 9d towards the connecting in-flow nozzle 22. Due to the geometric shape of the internal bore 9d, defined by the inner surface of the pin 9, including the ceiling 108 and the apertures (one shown for simplicity), the fluid contacts a relatively small amount of the pin's inner surface as it moves through the connector 1. In particular, the proportion of fluid which temporarily back-flows into the internal bore 9d due to contact with the ceiling 108 or the internal walls is minimised and the turbulence of flow inside the connector is thereby minimal.

Figures 5 and 5a show a variant of the pin 9 having a specifically shaped internal profile and aperture shape to maximise fluid flow rate. The hollow shaft 9a, having generally circular cross-section, extends through the centre of base 3. The shaft 9a comprises a relatively short lower shaft length 42 which extends below the base 3 and is engagable with the first fluid carrier when the base screw thread 13 and fluid carrier screw thread are in a mutual locking arrangement. The short length 42 has an internal diameter of about 2.9mm, allowing for a maximum flow rate through this part of the connector. The relatively longer length of the hollow shaft 9a, having an internal diameter of about 1.8mm, extends upwardly from the base 3 and gently tapers at its distal end, having there an internal diameter of about 1.0mm. The pin 9 comprises a tip portion 9b in the form of a solid cylindrical member that sits on the tapered end of the hollow shaft 9a. The wall of the tapered shaft 9a comprises three apertures 11a, 11b and 11c evenly spaced from one another around the circumference of the pin and directly below the solid cylindrical member 9b. The apertures 11 have a width of about 1.0mm and a length of about 2.0mm.

It is desired that the flow rate from the connector 1 into the first fluid carrier should be not less than 185ml per minute at 1 metre pressure (0.0098mPa). Testing by the Applicants has confirmed that a connector with a pin having at least two apertures achieves a flow rate in the range of 192.5ml to 341.7ml per minute. In a particularly preferred embodiment of the connector, where the connector comprises three apertures and includes the features described in Figures 3a, 3b and 4a, computational fluid dynamics studies have also predicted improved flow rates compared to the prior art. Flow rates in the preferred embodiment lie in the range of 236 to 279ml/min on injection and in the range of 200 to 296ml/min on aspiration. This is an improvement of flow rate as compared to two known devices currently in the market: Smart Site (Alaris Medical) - in the range of 168.8 to 190ml/min (confirmed by testing in 2007 and 2008); and Clave (ICU Medical) - 165ml/min (as per their technical data sheet).

The Applicants have demonstrated during experimental bolus testing that in the particularly preferred embodiment of the invention the volume of fluid displaced during disconnection of the connector is in the range of -0.001 cc to -0.005cc. This is an improvement as compared to the bolus volumes of Smart Site (-0.026cc to - 0.045cc), ICU (-0.008cc to -0.030cc) and Halkey Roberts (-0.010cc to -0.025cc).

The Applicants have demonstrated during experimental bolus testing that in the particularly preferred embodiment of the invention the volume of fluid displaced on connection of the connector is in the range of 0.012cc and 0.015cc. This is an improvement as compared to the bolus volumes of Smart Site (-0.043cc to - 0.046cc), ICU (-0.012cc to -0.035cc) and Halkey Roberts (-0.017cc to -0.033cc).

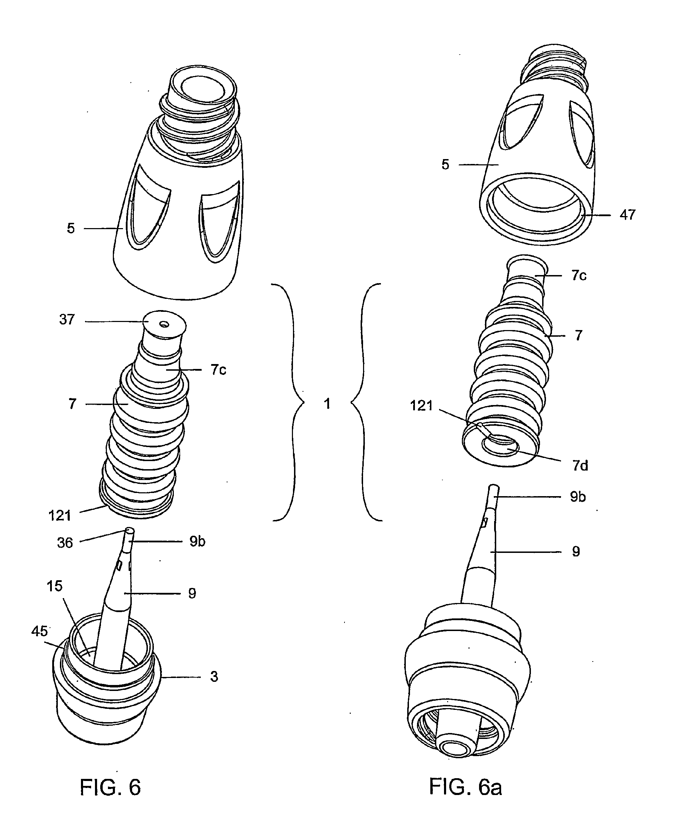

Referring to Figure 6 and 6a there is shown two views of an exploded perspective arrangement of the components of the connector 1 according to the embodiment previously described in Figures 3a, 3b, 4a, 5 and 5a including a substantially hollow pin 9 and integrated base 3, a resilient member 7 and a housing 5. The base 3 and housing 5 are typically made from polycarbonate material.

The connector 1 is therefore made of three components in total, the pin 9-base 3, the resilient member 7 and the housing 5 which allows the connector 1 to be assembled with limited technical skill. Moreover, the simplistic design of each of the three components results in a connector which is easily manufactured and which may be taken apart and re-constructed with ease should any part of the connector need replacing.

The resilient member 7 is typically made of an elastomeric material, such as silicone, and is generally hollow in structure, the sealing end 7c having a central cylindrical bore 7d there through with a diameter of about 0.8mm. The substantially hollow pin 9 comprises a solid cylindrical member 9b having a diameter of 1.0mm. The resilient member 7 is mounted about the pin 9, the diameter of the cylindrical bore 7d being stretched to accommodate the diameter of solid cylindrical member 9b. Upper surface 37 of the sealing end 7c is arranged to lie flush with an upper surface 36 of solid cylindrical member 9b to create a flat unbroken receiving surface.

The base 3 has a support element 15 in the form of a recess which is able to receive and securely hold the bottom part 7a. Figure 6a clearly shows the underside of the bottom part 7a of the resilient member 7. This bottom part 7a comprises a recess or channel formation 121 which defines an air pathway between the inside of the resilient member 7 and the outside when the connector 1 is assembled. Any air which is trapped inside the resilient member 7 may move through to the outside thereof when the connector 1 is in use.

Housing 5 is positioned over the resilient member 7 and rim 47 is secured to lip 45 of base 3 by means of ultra sonic welding. In this state, a longitudinally contractible portion 7b of the resilient member 7 is very lightly compressed by virtue of the surrounding load of the neck housing 5a. As a result, when a nozzle is pressed downwardly on to the tip portion 7c, the tip portion 7c simultaneously slides down the pin. No preliminary displacement of the resilient member occurs prior to compression of the resilient member 7. This is also a useful component design when considering manufacturing tolerances. However, the resilient member 7 is still in a generally non-compressed state relative to its compressed state when the nozzle has fully engaged the connector 1.

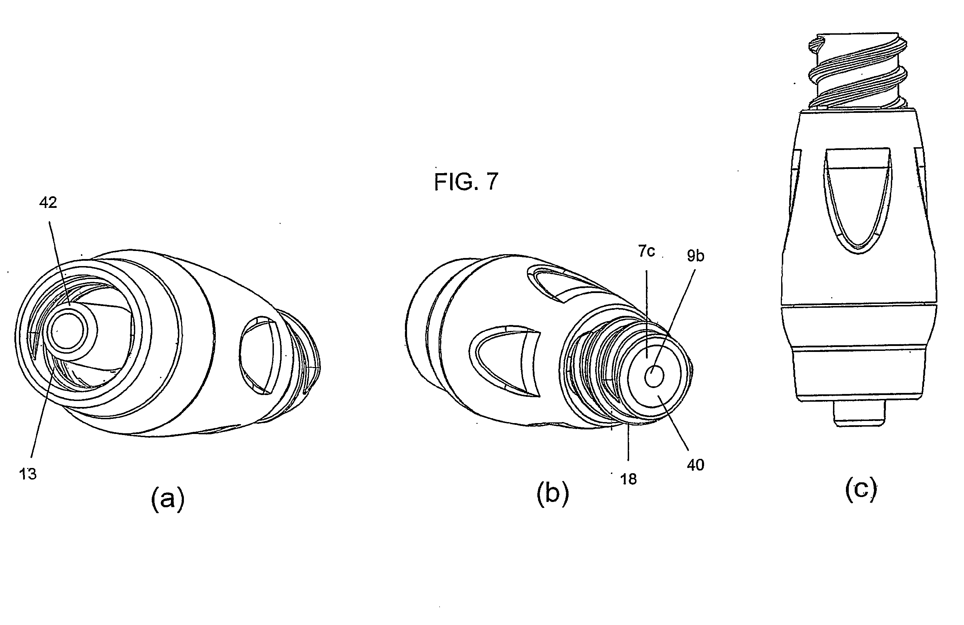

Figure 7 has three different viewing angles (a), (b) and (c), showing the general design concept of connector 1. Connector 1 is illustrated having base 3 (and pin 9) locked in a fitting arrangement with housing 5, resilient member 7 being securely positioned there between.

More particularly, (a) is a perspective view of the connector 1 showing the length 42 of the pin shaft 9a extending outwardly from the base 3 and along a central longitudinal axis of the connector. As can be seen, the thread lock 13 surrounds length 42 such that the connector may be securely connected with a fluid carrier having a complementary thread configuration. (b) is a perspective view of the connector 1 showing the receiving surface 40 defined by the surfaces of the cylindrical member 9b of the pin and tip portion 7c of the resilient member. The neck of housing 5a has threads 18 which enable the housing 5 to be secured to a fluid carrier with a corresponding thread lock configuration. Finally (c) is a side view of the connector 1 in an upright position.

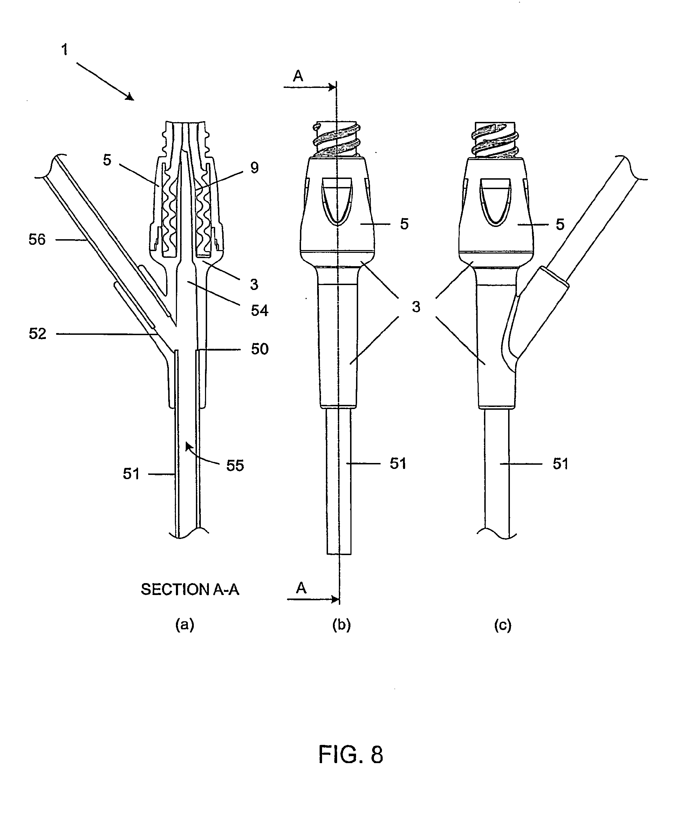

Figure 8 shows three different views (a), (b) and (c) of an embodiment of the connector 1 having a base 3 and integrated pin 9 and a housing 5. In this particular embodiment of the invention, the base 3 does not require a screw thread or luer lock arrangement for the connector 1 to be connected with a fluid carrier.

View (a) shows a cross section of the connector 1 taken along its longitudinal axis (length of the base 3, pin 9 and housing 5 together) shown as line "A-A" in View (b). The base 3 is completely integrated with the pin 9. Here, a lower shaft 54 of the base 3 replaces a lower shaft of the pin (not shown) that in other embodiments extends a short way down from the base 3. The lower shaft 54 and a cylindrical passage 50 of the base 3, together with a shaft of the pin 9, define the main fluid pathway 55 which runs axially through the connector. The first cylindrical passage 50 is connected with a fluid carrier such as tubing 51 or the like. Tubing 51 of an appropriate diameter is inserted within the cylindrical passage 50 so that the connector 1 is securely fitted to the tubing 51 and fluid is able to be carried to or from the connector 1. The base 3 further defines a second cylindrical passage 52 which extends outwardly from the first cylindrical passage 50 providing a second connection point with the base 3 of the connector 1. The second cylindrical passage 52 also receives a section of tubing 56, for example, as described with respect to the first passage 50. The main fluidic pathway 55 running through the connector 1 is accessible directly by connecting a tube 56 to the second passage 52, without interfering with the existing physical connection at the first passage 50.

For simplicity, the first and second passages 50, 52 of the base 3 are shown with a similar diameter, length and cross-sectional shape. However, it is envisaged by the inventors that the first and second cylindrical passages 50, 52 may not be identical in size or shape, but may be manufactured to provide a secure fit with fluid carriers of a commercially desirable size and shape. In the embodiment depicted, the branched part 52 extends generally obliquely towards the pin 9 relative to the connector's longitudinal axis A-A.

This embodiment of the connector 1 also shares the preferred features of the resilient member 7 and the housing 5, previously described with reference to Figure 3b, for example.

A perspective view (b) shows the whole connector 1 turned clockwise at a 90 degree angle from its original position in view (a). The tubing 51 is fitted inside the first passage (not shown) of the base 3. View (c) shows an additional view where the connector has been turned clockwise through a further 90 degrees.

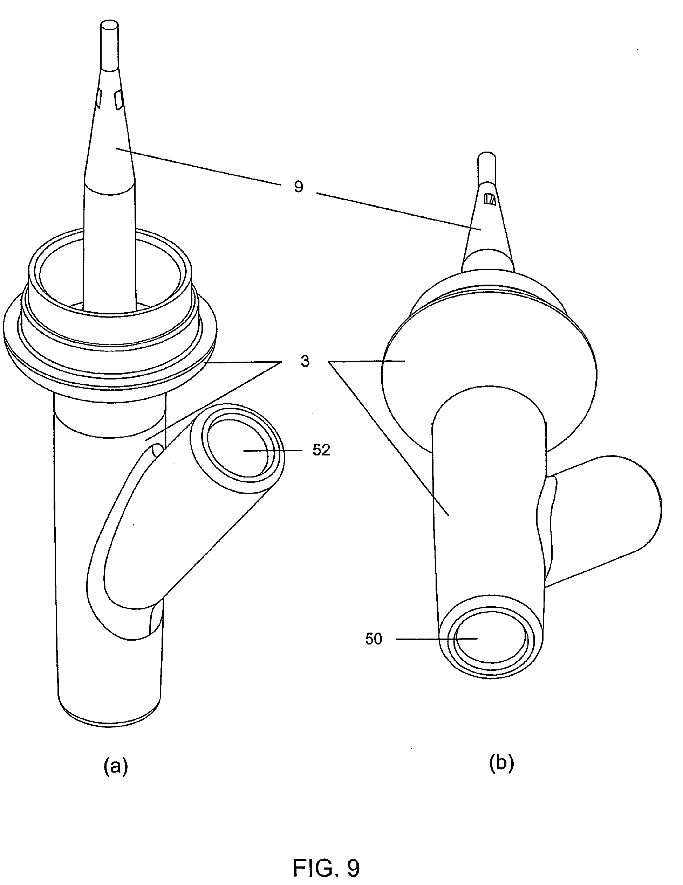

Figure 9 shows perspective views (a) and (b) of the embodiment described in Figure 8. The housing and resilient member of the connector have been removed to provide a clear view of the pin 9 in relation to the base 3. The pin 9 is shown extending up from base 3, the base 3 having first and second passages 50 and 52 arranged as previously described with reference to Figure 8.

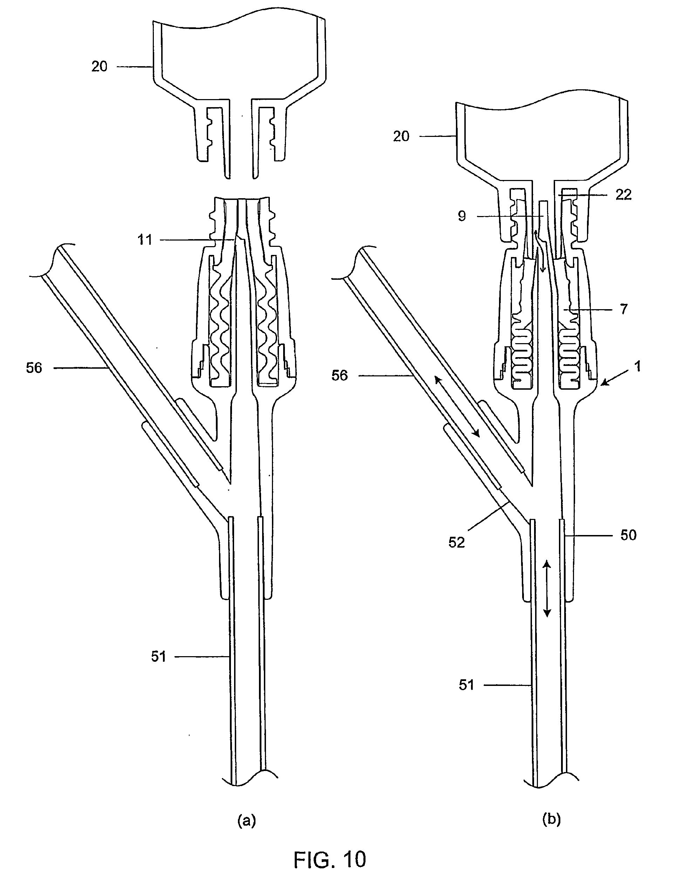

Figure 10 shows two views of the same connector 1 as Figure 8. Additionally, this Figure shows the connector prior to and during engagement with fluid carrier 20 at its pin end, the mechanism of which was previously described with reference to Figure 3b. For ease of comparison, Figure 10 comprises two adjacent cross-sectional views of the connector 1, of which, view (a) is an enlarged view (a) of Figure 8 and view (b) shows the connector 1 and its pin 9 engaged with a nozzle 22 of a fluid carrier 20. Thus, three connections in total are shown: the pin 9 end of the connector 1 (by virtue of the position of the resilient member 7 providing access to the aperture(s) 11) engaged with the nozzle 22 of fluid carrier 20; and at the base 3 at both the first and second passages 50, 52, being connected to tubing 51, 56, respectively.

## Claims

1. A self-sealing connector (1) for establishing fluidic communication between two fluid carrier lines, the connector (1) comprising:
a housing (5) including a neck;
a base (3);
a substantially hollow pin (9) extending up through said base (3) into said housing (5), the hollow pin (9) having a tip portion (9b) and at least one aperture (1); and a resilient member (7) mounted about said pin (9), the resilient member (7) having a sealing end (7c) for sealing the at least one aperture (11), **characterised in that** a surface of the sealing end (7c) and a surface of the tip portion (9b) of the pin (9) together defining a planar receiving surface such as for a nozzle (22), and **in that** the sealing end (7c) of the resilient member (7) further includes a flange seal (26) adapted to maintain sealing contact with an inner surface of the housing neck (5a) both when the resilient member (7) is in a first position and the at least one aperture (11) is sealed and when the resilient member (7) is compressed to allow fluid to flow through the at least one aperture (11).

2. The self-sealing connector (1) of Claim 1, wherein an inner surface of the sealing end (7c) contacts the surface of the tip portion (9b) when the resilient member (7) is in the first position.

3. The self-sealing connector (1) of Claim 2, wherein the sealing end (7c) contacts the tip portion (9b) of the pin (9), above and below the at least one aperture (11).

4. The self-sealing connector (1) of Claim 3, wherein the sealing end (7c) comprises two sealing rings (100, 102) for contacting the pin (9).

5. The self-sealing connector (1) of any preceding claim, wherein the at least one aperture (11) is proximal to the tip portion (9b).

6. The self-sealing connector (1) of any preceding claim, wherein when the resilient member (7) is in a substantially non-compressed state, the sealing end (7c) is adapted to extend from the tip portion (9b) to below the at least one aperture (11).

7. The self-sealing connector (1) of any preceding claim, wherein the neck (5a) of the housing (5) and the sealing end (7c) are substantially the same length

8. The self-sealing connector (1) of any preceding claim, wherein, in use, the sealing end (7c) is adapted to circumferentially seal around the hollow pin (9) below the at least one aperture (11) when the resilient member (7) is axially compressed below the tip portion (9b) of the pin (9) in the direction of the base (3).

9. The self-sealing connector (1) of any preceding claim, wherein the resilient member (7) comprises a bottom flange having a channel (121) therein for communicating the inside of the resilient member (7) with the outside thereof.

10. The self-sealing connector (1) of any preceding claim, wherein the substantially hollow pin (9) has at least two apertures (11).

11. The self-sealing connector (1) of any preceding claim, wherein the substantially hollow pin (9) has three apertures (11a, 11b, 11c).

12. The self-sealing connector (1) of Claim 10 or 11, wherein the apertures (11 or 11 a, 11 b, 11c) are equally radially spaced from one another.

13. The self-sealing connector (1) of any preceding claim, wherein the aperture(s) (11) extend obliquely through the wall of the pin (9).

14. The self-sealing connector (1) of any preceding claim, wherein a shaft of the pin (9d) has a convex internal ceiling (108).

15. The self-sealing connector (1) of any preceding claim, wherein the base (3) comprises a first passage (50) arranged to make a connection with tubing (51) or the like and a second passage (52) branching out from said first passage (50) such that access to a pathway of the connector is enabled independently from connection at the first passage (50) of the base (3).

## Patentansprüche

1. Selbstdichtender Verbinder (1) zum Herstellen von Fluidverbindung zwischen zwei Fluidträgerleitungen, wobei der Verbinder (1) Folgendes umfasst:
ein Gehäuse (5) mit einem Hals;
eine Basis (3);
einen im Wesentlichen hohlen Stift (9), der sich durch die Basis (3) nach oben in das Gehäuse (5) erstreckt, wobei der hohle Stift (9) einen Spitzenabschnitt (9b) und mindestens eine Öffnung (1) aufweist; und ein um den Stift (9) angebrachtes elastisches Element (7), wobei das elastische Element (7) ein Dichtungsende (7c) zum Dichten der mindestens einen Öffnung (11) aufweist, **dadurch gekennzeichnet, dass** eine Fläche des Dichtungsendes (7c) und eine Fläche des Spitzenabschnitts (9b) des Stifts (9) zusammen eine ebene Aufnahmefläche, wie etwa für eine Düse (22) definieren, und dadurch, dass das Dichtungsende (7c) des elastischen Elements (7) weiter eine Flanschdichtung (26) umfasst, die dazu angepasst ist, sowohl wenn sich das elastische Element (7) in einer ersten Position befindet und die mindestens eine Öffnung (11) gedichtet ist als auch, wenn das elastische Element (7) zusammengedrückt ist, um Fluid durch die mindestens eine Öffnung (11) fließen zu lassen, dichtenden Kontakt mit einer Innenfläche des Gehäusehalses (5a) aufrechtzuerhalten.

2. Selbstdichtender Verbinder (1) nach Anspruch 1, wobei eine Innenfläche des Dichtungsendes (7c) mit der Fläche des Spitzenabschnitts (9b) in Kontakt steht, wenn sich das elastische Element (7) in der ersten Position befindet.

3. Selbstdichtender Verbinder (1) nach Anspruch 2, wobei das Dichtungsende (7c) oberhalb und unterhalb der mindestens einen Öffnung (11) mit dem Spitzenabschnitt (9b) des Stifts (9) in Kontakt steht.

4. Selbstdichtender Verbinder (1) nach Anspruch 3, wobei das Dichtungsende (7c) zwei Dichtungsringe (100, 102) umfasst, um mit dem Stift (9) in Kontakt zu treten.

5. Selbstdichtender Verbinder (1) nach einem der vorangehenden Ansprüche, wobei die mindestens eine Öffnung (11) proximal zu dem Spitzenabschnitt (9b) ist.

6. Selbstdichtender Verbinder (1) nach einem der vorangehenden Ansprüche, wobei, wenn sich das elastische Element (7) in einem im Wesentlichen nicht zusammengedrückten Zustand befindet, das Dichtungsende (7c) dazu angepasst ist, sich von dem Spitzenabschnitt (9b) bis unterhalb der mindestens einen Öffnung (11) zu erstrecken.

7. Selbstdichtender Verbinder (1) nach einem der vorangehenden Ansprüche, wobei der Hals (5a) des Gehäuses (5) und das Dichtungsende (7c) im Wesentlichen gleich lang sind.

8. Selbstdichtender Verbinder (1) nach einem der vorangehenden Ansprüche, wobei in Gebrauch das Dichtungsende (7c) dazu angepasst ist, unterhalb der mindestens einen Öffnung (11) in Umfangsrichtung um den hohlen Stift (9) zu dichten, wenn das elastische Element (7) unterhalb des Spitzenabschnitts (9b) des Stifts (9) in Richtung der Basis (3) axial zusammengedrückt ist.

9. Selbstdichtender Verbinder (1) nach einem der vorangehenden Ansprüche, wobei das elastische Element (7) einen unteren Flansch mit einer Rinne (121) darin zum Verbinden des Inneren des elastischen Elements (7) mit dem Äußeren desselben umfasst.

10. Selbstdichtender Verbinder (1) nach einem der vorangehenden Ansprüche, wobei der im Wesentlichen hohle Stift (9) mindestens zwei Öffnungen (11) aufweist.

11. Selbstdichtender Verbinder (1) nach einem der vorangehenden Ansprüche, wobei der im Wesentlichen hohle Stift (9) drei Öffnungen (11a, 11 b, 11 c) aufweist.

12. Selbstdichtender Verbinder (1) nach Anspruch 10 oder 11, wobei die Öffnungen (11 bzw. 11a, 11 b, 11 c) in gleichen radialen Abständen voneinander angeordnet sind.

13. Selbstdichtender Verbinder (1) nach einem der vorangehenden Ansprüche, wobei sich die Öffnung(en) (11) schräg durch die Wand des Stifts (9) erstreckt bzw. erstrecken.

14. Selbstdichtender Verbinder (1) nach einem der vorangehenden Ansprüche, wobei ein Schaft des Stifts (9d) eine konvexe innere Decke (108) aufweist.

15. Selbstdichtender Verbinder (1) nach einem der vorangehenden Ansprüche, wobei die Basis (3) einen ersten Durchgang (50), der dazu angeordnet ist, eine Verbindung mit Röhrenmaterial (51) oder dergleichen herzustellen und einen zweiten Durchgang (52), der von dem ersten Durchgang (50) abzweigt, umfasst, so dass Zugang zu einem Leitungsweg des Verbinders unabhängig von der Verbindung an dem ersten Durchgang (50) der Basis (3) ermöglicht ist.

## Revendications

1. Connecteur auto-obturateur (1) pour établir une communication fluidique entre deux conduites porteuses de fluide, le connecteur (1) comprenant :
un boîtier (5) comportant un col ;
une base (3) ;
une broche sensiblement creuse (9) s'étendant à travers ladite base (3) jusque dans ledit boîtier (5), la broche creuse (9) ayant une partie de pointe (9b) et au moins une ouverture (1) ; et un élément élastique (7) monté autour de ladite broche (9), l'élément élastique (7) ayant une extrémité obturatrice (7c) pour obturer l'au moins une ouverture (11), **caractérisé en ce qu'**une surface de l'extrémité obturatrice (7c) et une surface de la partie de pointe (9b) de la broche (9) définissent ensemble une surface réceptrice plane telle que pour une buse (22), et **en ce que** l'extrémité obturatrice (7c) de l'élément élastique (7) comporte en outre une bride d'obturation (26) adaptée pour maintenir un contact d'obturation avec une surface interne du col de boîtier (5a) à la fois quand l'élément élastique (7) se trouve dans une première position et l'au moins une ouverture (11) est obturée et quand l'élément élastique (7) est compressé pour permettre au fluide de s'écouler à travers l'au moins une ouverture (11).

2. Connecteur auto-obturateur (1) selon la revendication 1, dans lequel une surface interne de l'extrémité obturatrice (7c) fait contact avec la surface de la partie de pointe (9b) quand l'élément élastique (7) se trouve dans la première position.

3. Connecteur auto-obturateur (1) selon la revendication 2, dans lequel l'extrémité obturatrice (7c) fait contact avec la partie de pointe (9b) de la broche (9), au-dessus et en dessous de l'au moins une ouverture (11).

4. Connecteur auto-obturateur (1) selon la revendication 3, dans lequel l'extrémité obturatrice (7c) comprend deux anneaux obturateurs (100, 102) pour faire contact avec la broche (9).

5. Connecteur auto-obturateur (1) selon l'une quelconque des revendications précédentes, dans lequel l'au moins une ouverture (11) est proche de la partie de pointe (9b).

6. Connecteur auto-obturateur (1) selon l'une quelconque des revendications précédentes, dans lequel l'élément élastique (7) se trouve dans un état sensiblement non compressé, l'extrémité obturatrice (7c) est adaptée pour s'étendre depuis la partie de pointe (9b) jusqu'à en dessous de l'au moins une ouverture (11).

7. Connecteur auto-obturateur (1) selon l'une quelconque des revendications précédentes, dans lequel le col (5a) du boîtier (5) et l'extrémité obturatrice (7c) sont sensiblement de la même longueur.

8. Connecteur auto-obturateur (1) selon l'une quelconque des revendications précédentes, dans lequel, durant l'utilisation, l'extrémité obturatrice (7c) est adaptée pour obturer circonférentiellement le pourtour de la broche creuse (9) en dessous de l'au moins une ouverture (11) quand l'élément élastique (7) est compressé axialement en dessous de la partie de pointe (9b) de la broche (9) dans le sens de la base (3).

9. Connecteur auto-obturateur (1) selon l'une quelconque des revendications précédentes, dans lequel l'élément élastique (7) comprend une bride inférieure comportant un canal (121) permettant à l'intérieur de l'élément élastique (7) de communiquer avec l'extérieur de celui-ci.

10. Connecteur auto-obturateur (1) selon l'une quelconque des revendications précédentes, dans lequel la broche sensiblement creuse (9) comporte au moins deux ouvertures (11).

11. Connecteur auto-obturateur (1) selon l'une quelconque des revendications précédentes, dans lequel la broche sensiblement creuse (9) comporte trois ouvertures (11 a, 11b, 11c).

12. Connecteur auto-obturateur (1) selon la revendication 10 ou 11, dans lequel les ouvertures (11 ou 11a, 11 b, 11 c) sont radialement équidistantes les unes des autres.

13. Connecteur auto-obturateur (1) selon l'une quelconque des revendications précédentes, dans lequel la ou les ouvertures (11) s'étendent obliquement à travers la paroi de la broche (9).

14. Connecteur auto-obturateur (1) selon l'une quelconque des revendications précédentes, dans lequel un axe de la broche (9d) a un plafond interne convexe (108).

15. Connecteur auto-obturateur (1) selon l'une quelconque des revendications précédentes, dans lequel la base (3) comprend un premier passage (50) agencé pour établir une connexion avec un tubage (51) ou assimilé et un second passage (52) émanant dudit premier passage (50) de façon à permettre un accès à un passage du connecteur indépendamment de la connexion au niveau du premier passage (50) de la base (3).
